(19) Europäisches Patentamt
European Patent Office
Office européen des brevets

(11) EP 1 510 221 A1

(12) **EUROPEAN PATENT APPLICATION**
published in accordance with Art. 158(3) EPC

(43) Date of publication:
**02.03.2005 Bulletin 2005/09**

(21) Application number: **03733264.0**

(22) Date of filing: **03.06.2003**

(51) Int Cl.[7]: **A61K 45/00**, A61K 31/517, A61K 31/519, A61K 31/5377, A61P 9/10, A61P 17/06, A61P 27/02, A61P 35/00

(86) International application number:
**PCT/JP2003/006988**

(87) International publication number:
**WO 2003/101491 (11.12.2003 Gazette 2003/50)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **03.06.2002 JP 2002162130**

(71) Applicant: **Mitsubishi Pharma Corporation**
**Osaka-shi, Osaka 541-0046 (JP)**

(72) Inventors:
- **SUZUKI, Tsuyoshi,**
**MITSUBISHI PHARMA CORPORATION**
**Chuo-ku, Tokyo 103-8405 (JP)**
- **KITANO, Yasunori,**
**MITSUBISHI PHARMA CORPORATION**
**Chuo-ku, Tokyo 103-8405 (JP)**
- **YANO, Shinji,**
**MITSUBISHI PHARMA CORPORATION**
**Chuo-ku, Tokyo 103-8405 (JP)**

(74) Representative:
**von Kreisler, Alek, Dipl.-Chem. et al**
**Deichmannhaus am Dom,**
**Postfach 10 22 41**
**50462 Köln (DE)**

(54) **PREVENTIVES AND/OR REMEDIES FOR SUBJECTS WITH THE EXPRESSION OR ACTIVATION OF HER2 AND/OR EGFR**

(57) A Her2 and/or EGFR inhibitor to be administered to a subject determined to show overexpression or activation of Her2 and/or EGFR as a result of a diagnosis of the subject for the expression or activity of Her2 and/or EGFR based on a test for detecting the expression or activity of Her2 and/or EGFR, and a pharmaceutical composition containing the inhibitor.

FIG. 1

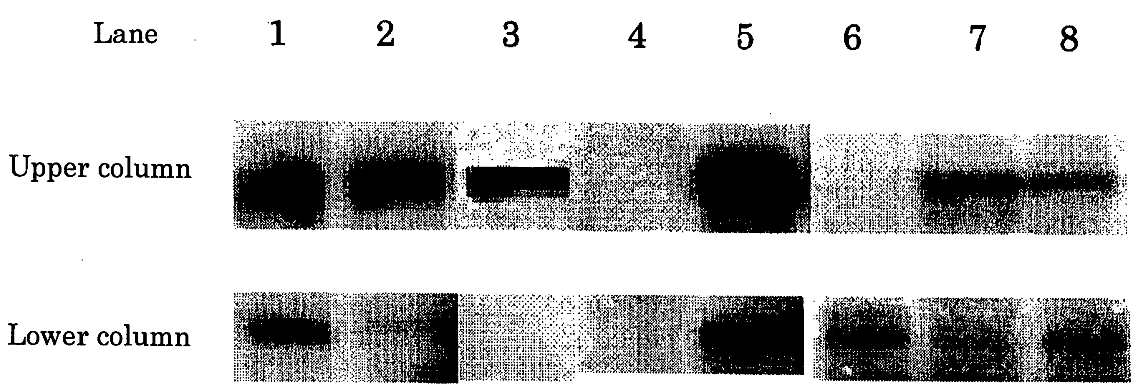


EP 1 510 221 A1

Printed by Jouve, 75001 PARIS (FR)

## Description

## Technical Field

The present invention relates to a pharmaceutical agent to be used for a series of treatment methods comprising diagnosing a subject for expression or activation of Her2 and/or EGFR and treating the subject confirmed to show expression thereof.

## Background Art

Conventionally, the target molecules of anti-cancer agents have been mainly those related with DNA or RNA synthesis and cell division. Such anti-cancer agents are known to cause severe side effects such as bone marrow toxicity and the like, because the target molecules are not specific for cancer cells.

With the development of molecular oncology in recent years, it has been clarified that cancer is induced by abnormality of oncogenes or tumor suppressor genes. As the most well known oncogenes, epithelial growth factor receptor (hereinafter to be abbreviated as EGFR) and analogous human EGFR type 2 (hereinafter to be abbreviated as Her2, different name, also referred to as erbB-2) can be mentioned.

EGFR and Her2 are both transmembrane glycoproteins respectively having a molecular weight of 170kD, 185kD. A tyrosine kinase domain is present in the intracellular domains of EGFR and Her2, and transduct signals to the nucleus via phosphorylation reactions. The amino acid sequences of kinase domain have about 80% of homology and are structurally highly similar. However, the homology of autophosphorylation domain in the C-terminal is as low as about 30%, which suggests qualitative difference in the signal transduction mechanisms of the both receptors.

EGFR expresses in the esophageal cancer at an extremely high frequency of 89% [J. Cancer Res. Clin. Oncol. 1993 119, 401-407]. In other cancers, it has been reported that an overexpression of EGFR is found in 45% of non-small cell lung cancer [Cancer Res. 1993 53, 2379-2385], gene amplification of EGFR in 50% of glioblastoma [Cancer Res. 2000 60, 1383-1387] and the like.

As for Her2, gene amplification is observed in 39% of breast cancer [J. Clin. Oncol. 1993 11, 1936-1942], overexpression is observed in 32% of ovarian cancer [Cancer Res. 1990 50, 4087-4091], overexpression is observed in 67% of hormone-resistant prostate cancer [Clinical Cancer Res. 2001 7, 2643-2647] and the like.

While EGFR and Her2 are over-expressed in various cancers, the substantial molecular mechanism of tumorigenesis by them is a signal transduction via a phosphorylation reaction, and the extensive activation of signal transduction may clinically deteriorate cancer progression independently of the level of expression amount of EGFR and Her2. Therefore, as long as the target expression varies depending on the cancers, a treatment targeting these molecules require individual treatments, wherein the nature of the cancer should be diagnosed, and a pharmaceutical agent should be determined depending on the presence or absence of expression and activation of the target molecules. This affords an effective and reasonable treatment.

EGFR and Her2 form a heterocomplex and shows functional interaction [J. Clin. Oncol. 2001 19(18s), 32s-40s]. It is known that coexpression of EGFR and Her2 accelerates tumorigenesis by EGFR alone [Cell 1987 58, 287-292]. In addition, there are reports that coexpression of EGFR and Her2 in breast cancer, oral cavity cancer, lung cancer and the like leads to poor prognosis [Clin. Cancer Res. 1999 5, 4164-4174].

Therefore, a pharmaceutical agent inhibiting both EGFR and Her2 (hereinafter also referred to simply as a dual inhibitor) is advantageous in that it is applicable to a wider range of diseases as compared to a pharmaceutical agent acting only on either of them, and also superior in that it has a potential to provide an effective treatment of coexpressed cancers based on a synergistic action of dual inhibition.

The diagnostic techniques of EGFR and Her2 are already widely known. A recent report has documented on an attempt to detect a precancer lesion of oral cavity cancer by conjugating a fluorescence dye to an antibody binding to EGFR [Cancer Res. 2001 61, 4490-4496]. In addition, a report has also appeared that suggests that an antitumor effect on anthracycline anti-cancer agents can be predicted by the presence or absence of Her2 expression in breast cancer patients [Clin. Cancer Res. 2001 7, 1577-1581]. Therefore, diagnosis of EGFR and Her2 may be useful for both the early discovery of disease and determination of pharmaceutical agents effective and suitable for individual patients.

As an EGFR inhibitor, neutralizing antibody and tyrosine kinase inhibitor are in the stage of clinical development for anti-cancer agents. As Her2 inhibitor, a neutralizing antibody Herceptin (product name, Roche) has been already marketed in various countries as a therapeutic agent for metastatic breast cancer. However, tyrosine kinase inhibitor is in the stage of clinical development for anti-cancer agent.

Similarly, a dual inhibitor of EGFR and Her2 has been studied and developed but has not been put to practical use.

The anti-Her2 antibody Herceptin has been already subjected to the patient selection with Her2 expression

using diagnosis Herceptest (product name). Its indication is limited to breast cancer.

An EGFR kinase inhibitor Iressa (product name, A. Zeneca) has been proven clinically to cause no severe side effects that conventional anti-cancer agents have, such as bone marrow toxicity and the like, and to be effective for non-small cell lung cancer [Clin. Cancer Res. 2001 7, 3780s]. In clinical studies for Iressa, however, prior confirmation of EGFR expression was not performed for selection of patients.

A dual inhibitor of EGFR and Her2 can be applied to a wide range of cancers, because it can be applied as long as either target molecule is expressed therein. As mentioned earlier, however, prescription for patients, which are appropriately determined based on the diagnosis of expression or activation of target protein, is desirable in a more precise sense, and this series of treatment methods using a dual inhibitor has not been established yet in clinical situations.

As a different example relating to the expression of target molecule in cancer tissues and the sensitivity of anti-cancer agents, a report has documented that the expression amount of thymidylate synthase (TS) and dihydropyrimidine dehydrogenase activity in breast cancer tissues; and 5FU sensitivity are related [Journal of Japan Society of Clinical Oncology, 2000 35, 340]. However, TS expression diagnosis has not yet led to be performed in fact as a treatment method before prescription of 5FU.

As mentioned above, it has been desired to predict an effective treatment method by diagnosing the target molecules of the pharmaceutical agents for prescription of anti-cancer agents.

**Disclosure of the Invention**

In view of the above-mentioned situation, the present inventors have conducted intensive studies in an attempt to solve the aforementioned problems and found that a series of treatment methods can be established by diagnosing a subject for expression or activity of Her2 and/or EGFR, and administering a Her2 and/or EGFR inhibitor to the subject confirmed to show the expression or activity.

Namely, the present invention relates to a Her2 and/or EGFR inhibitor to be administered in such series of treatment methods, a pharmaceutical composition containing the inhibitor as an active ingredient, and a prophylactic and/or therapeutic method comprising administering the inhibitor to a subject, and more particularly, the present invention provides the following.

The present invention relates to

(1) a Her2 and/or EGFR inhibitor to be administered to a subject determined to show overexpression or activation of Her2 and/or EGFR as a result of a diagnosis of the subject for the expression or activity of Her2 and/or EGFR based on a test for detecting the expression or activity of Her2 and/or EGFR,

(2) the inhibitor of the aforementioned (1) to be administered to a subject determined to show activation of Her2 and/or EGFR as a result of a diagnosis of the subject for the activity of Her2 and/or EGFR based on a test for detecting the activity of Her2 and/or EGFR,

(3) the inhibitor of the aforementioned (1), which is a Her2 and EGFR inhibitor for administration to a subject determined to show overexpression or activation of Her2 and EGFR as a result of a diagnosis of the subject for the expression or activity of Her2 and EGFR based on a test for detecting the expression or activity of Her2 and EGFR,

(4) the inhibitor of the aforementioned (3) to be administered to a subject determined to show activation of Her2 and EGFR as a result of a diagnosis of the subject for the activity of Her2 and EGFR based on a test for detecting the activity of Her2 and EGFR,

(5) the inhibitor of the aforementioned (1), wherein the subject is expected to suffer from a disease caused by overexpression or activation of Her2 and/or EGFR,

(6) the inhibitor of the aforementioned (3), wherein the subject is expected to suffer from a disease caused by overexpression or activation of Her2 and EGFR,

(7) the inhibitor of any of the aforementioned (1) to (6), wherein the subject is a human,

(8) the inhibitor of the aforementioned (1), wherein the test for detecting the expression or activity of Her2 and/or EGFR is an extracorporeal test,

(9) the inhibitor of the aforementioned (3), wherein the test for detecting the expression or activity of Her2 and EGFR is an extracorporeal test,

(10) the inhibitor of the aforementioned (3), which is a mixture of a Her2 inhibitor and an EGFR inhibitor,

(11) the inhibitor of the aforementioned (3), which is used for administering a Her2 inhibitor and/or an EGFR inhibitor simultaneously, separately or at time intervals,

(12) the inhibitor of the aforementioned (8) or (9), wherein the extracorporeal test is an immunological method using an antibody, or a hybridization method using a nucleic acid and a nucleic acid derivative,

(13) the inhibitor of the aforementioned (12), wherein the immunological method using an antibody is selected

from the group consisting of an enzyme-linked immunosorbent assay, an enzyme-linked immunoassay, a radioimmunoassay, an immunohistochemical method and western blotting,

(14) the inhibitor of the aforementioned (12), wherein the hybridization method using a nucleic acid and a nucleic acid derivative is selected from the group consisting of an RT-PCR method, an ISH method, a FISH method, northern blotting and southern blotting method,

(15) the inhibitor of any of the aforementioned (1) to (14), which is a substituted heteroaromatic compound represented by the following formula (I)

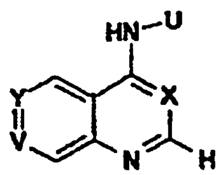 (I) 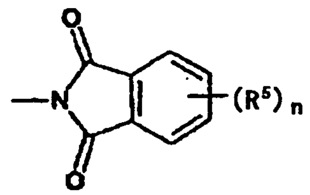 (a)

wherein X is N or CH; Y is $CR^1$ and V is N; or Y is N and V is $CR^1$; or Y is $CR^1$ and V is $CR^2$; or Y is $CR^2$ and V is $CR^1$; $R^1$ is $C_{1-4}$ alkyl, $C_{1-4}$ alkoxy, $CH_3SO_2CH_2CH_2NHCH_2$-Ar- (wherein Ar is selected from phenyl, furan, thiophene, pyrrole and thiazole, each of which is optionally substituted by 1 or 2 halogens, $C_{1-4}$ alkyl or $C_{1-4}$ alkoxy on demand) or -C=C-C ($R^6$) ($R^7$) ($R^8$) (wherein $R^6$, $R^7$ and $R^8$ are each independently a hydrogen atom, hydroxy, halogen, $C_{1-4}$ alkyl or $C_{1-4}$ alkoxy, or $C_{3-6}$ cycloalkyl wherein the ring is optionally substituted by hydrogen atom or $C_{1-4}$ alkyl and optionally contains 1 or 2 hetero atoms selected from O, S and N therein; $R^2$ is selected from the group consisting of hydrogen, halogen, hydroxy, $C_{1-4}$ alkyl, $C_{1-4}$ alkoxy, $C_{1-4}$ alkylamino, di [$C_{1-4}$ alkyl]amino and -NHCO-$R^9$ (wherein $R^9$ is $C_{1-4}$ alkyl, $C_{1-4}$ alkoxy, $C_{2-4}$ alkenyl or $C_{2-4}$ alkynyl) ; U is phenyl, pyridyl, 3H-imidazolyl, indolyl, isoindolyl, indolinyl, isoindolinyl, 1H-indazolyl, 2,3-dihydro-1H-indazolyl, 1H-benzimidazolyl, 2,3-dihydro-1H-benzimidazolyl or 1H-benzotriazolyl group, each of which is substituted by $R^3$ group and optionally substituted on demand by at least one $R^4$ group selected independently; $R^3$ is selected from the group consisting of benzyl, halo-, dihalo- and trihalobenzyl, benzoyl, pyridylmethyl, pyridylmethoxy, phenoxy, benzyloxy, halo-, dihalo- and tribenzyloxy and benzenesulfonyl; or $R^3$ is trihalomethylbenzyl or trihalomethylbenzyloxy; or $R^3$ is a group of the above-mentioned formula (a) (wherein each $R^5$ is independently selected from halogen, $C_{1-4}$ alkyl and $C_{1-4}$ alkoxy; and n is 0-3); each $R^4$ is independently hydroxy, halogen, $C_{1-4}$ alkyl, $C_{2-4}$ alkenyl, $C_{2-4}$ alkynyl, $C_{1-4}$ alkoxy, amino, $C_{1-4}$ alkylamino, di [$C_{1-4}$ alkyl]amino, $C_{1-4}$ alkylthio, $C_{1-4}$ alkylsulfinyl, $C_{1-4}$ alkylsulfonyl, $C_{1-4}$ alkylcarbonyl, carboxy, carbamoyl, $C_{1-4}$ alkoxycarbonyl, $C_{1-4}$ alkanoylamino, N- ($C_{1-4}$ alkyl) carbamoyl, N,N-di ($C_{1-4}$ alkyl) carbamoyl, cyano, nitro or trifluoromethyl, or a pharmaceutically acceptable salt thereof, a hydrate or solvate thereof, an optically active substance or a racemate thereof, or a mixture of diastereomers thereof,

(16) the inhibitor of any of the aforementioned (1) to (15), which is (4-(3-fluorobenzyloxy)-phenyl)-(6-(5-((2-methanesulfonyl-ethylamino)methyl)-furan-2-yl)-pyrido[3,4-d]pyrimidin-4-yl)-amine;
(4-benzyloxyphenyl)-(6-(5-((2-methanesulfonyl-ethylamino)methyl)-furan-2-yl)-quinazolin-4-yl)-amine;
N-{4-[(3-fluorobenzyl)oxy]phenyl}-6-[5-({[2-(methylsulfonyl)ethyl]amino}methyl)-2-furyl]-4-quinazolinamine;
N-[4-(benzyloxy)phenyl]-7-methoxy-6-[5-({[2-(methylsulfonyl)ethyl]amino}methyl)-2-furyl]-4-quinazolinamine;
N-(1-benzyl-1H-indazol-5-yl)-7-methoxy-6-[5-({[2-(methylsulfonyl)ethyl]amino}methyl)-2-furyl]-4-quinazolinamine;
N-{3-fluoro-4-[(3-fluorobenzyl)oxy]phenyl}-6-[5-({[2-(methylsulfonyl)ethyl]amino}methyl)-2-furyl]-4-quinazolinamine;
N-[1-(3-fluorobenzyl)-1H-indazol-5-yl]-6-[2-({[2-(methylsulfonyl)ethyl]amino}methyl)-1,3-thiazol-4-yl]-4-quinazolinamine;
6-[5-({[2-(methylsulfonyl)ethyl]amino}methyl)-2-furyl]-N-[4-(phenylsulfonyl)phenyl]-4-quinazolinamine;
N-{3-fluoro-4-[(3-fluorobenzyl)oxy]phenyl}-6-[2-({[2-(methylsulfonyl)ethyl]amino}methyl)-1,3-thiazol-4-yl]-4-quinazolinamine;
N-(1-benzyl-1H-indazol-5-yl)-6-[2-({[2-(methylsulfonyl)ethyl]amino}methyl)-1,3-thiazol-4-yl]-4-quinazolinamine;
N-(3-fluoro-4-benzyloxyphenyl)-6-[5-({[2-(methylsulfonyl)ethyl]amino}methyl)-4-furyl]-4-quinazolinamine;
N-(3-chloro-4-benzyloxyphenyl)-6-[2-({[2-(methylsulfonyl)ethyl]amino}methyl)-4-furyl]-4-quinazolinamine;
N-{3-chloro-4-[(3-fluorobenzyl)oxy]phenyl}-6-[5-({[2-(methylsulfonyl)ethyl]amino}methyl)-2-furyl]-4-quinazolinamine;
N-(1-benzyl-1H-indazol-5-yl)-7-fluoro-6-[5-({[2-(methylsulfonyl)ethyl]amino}methyl)-2-furyl]-4-quinazolinamine;
N-(3-trifluoromethyl-4-benzyloxyphenyl)-6-[5-({[2-(methylsulfonyl)ethyl]amino}methyl)-4-furyl]-4-quinazolinamine;

N-[4-(3-chloro-4-fluorophenyl)amino-7-[3-(4-morpholinyl)propoxy]quinazolin-6-yl]acrylamide;
N-{4-[(3-chloro-4-fluorophenyl)amino]-7-[3-methyl-3-(4-methyl-1-piperazinyl)-1-butynyl]-6-quinazolinyl}acrylamide; or
N-{3-chloro-4-[(3-fluorobenzyl)oxy]phenyl}-6-[5-({[2-(methanesulfonyl)ethyl]amino}methyl)-2-furyl]-4-quinazolinamine, or a pharmaceutically acceptable salt thereof, a hydrate or a solvate thereof, an optically active substance or a racemate thereof, or a mixture of diastereomers thereof,

(17) the inhibitor of any of the aforementioned (1) to (16), which is N-[4-(3-chloro-4-fluorophenyl)amino]-7-[3-(4-morpholinyl)propoxy]quinazolin-6-yl]acrylamide, or N-{3-chloro-4-[(3-fluorobenzyl)oxy]phenyl}-6-[5-({[2-(methanesulfonyl)ethyl]amino}methyl)-2-furyl]-4-quinazolinamine or a pharmaceutically acceptable salt thereof, a hydrate or a solvate thereof, an optically active substance or a racemate thereof, or a mixture of diastereomers thereof,

(18) a pharmaceutical composition comprising an inhibitor of any of the aforementioned (1) to (17) as an active ingredient and a pharmaceutically acceptable carrier,

(19) the pharmaceutical composition of the aforementioned (18), which is an agent for the prophylaxis and/or treatment of a disease caused by overexpression or activation of Her2 and/or EGFR,

(20) the pharmaceutical composition of the aforementioned (19), wherein the disease caused by the overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis,

(21) an agent for the prophylaxis and/or treatment of a disease caused by overexpression or activation of Her2 and/or EGFR, which is to be administered to a subject determined to show overexpression or activation of Her2 and/or EGFR as a result of a diagnosis of the subject for the expression or activity of Her2 and/or EGFR based on a test for detecting the expression or activity of Her2 and/or EGFR,

(22) the agent of the aforementioned (21), wherein the disease caused by overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis,

(23) a method for the prophylaxis and/or treatment of a disease caused by overexpression or activation of Her2 and/or EGFR, which comprises administering an effective dose of a Her2 and/or an EGFR inhibitor to a subject determined to show overexpression or activation of Her2 and/or EGFR as a result of a diagnosis of the subject for the expression or activity of Her2 and/or EGFR based on a test for detecting the expression or activity of Her2 and/or EGFR,

(24) the method of the aforementioned (23), wherein the disease caused by overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis,

(25) a commercial package comprising the pharmaceutical composition of any of the aforementioned (18) to (20) and a written matter associated therewith, the written matter stating that the pharmaceutical composition can or should be used for the prophylaxis and/or treatment of a disease caused by overexpression or activation of Her2 and/or EGFR, and

(26) the commercial package of the aforementioned (25), wherein the disease caused by overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis.

The respective definitions in the present invention are as follows.

As the "test for detecting expression of Her2 and/or EGFR", an immunological method using an antibody against Her2 and/or EGFR or a hybridization method using a nucleic acid and a nucleic acid derivative can be mentioned. More preferable specific examples of the immunological method include an enzyme-linked immunosorbent assay, an enzyme-linked immunoassay, a radioimmunoassay, an immunohistochemical method, western blotting and the like, and more preferable specific examples of the hybridization method include an RT-PCR method, an ISH method, a FISH method, northern blotting, southern blotting and the like.

The "enzyme-linked immunosorbent assay (ELISA)" is an enzyme-linked immunoassay (EIA) performed on a solid phase, which comprises labeling an antigen or antibody with an enzyme via a covalent bond and performing an enzyme-linked immunoassay for detecting the presence, on a solid phase, of the antibody or antigen utilizing the enzyme activity. This method is a radioimmunoassay (RIA) developed by E. Engval et al. in 1971, except that a radioisotope with which to label either antigen or antibody is replaced by a nonradioactive enzyme. In 1990, it was developed as a method also capable of measuring an antigen of a zeptomole ($10^{-21}$ mol) level.

The ELISA method includes direct antibody method, indirect antibody method, competitive method, two antibody sandwich method and the like, and a method suitable for assay object is selected. As the solid phase, agarose, microtiter well, latex particles and the like are used, as the labeling enzyme, peroxidase derived from horseradish is most frequently used. As other enzymes, alkaliphosphatase, galactosidase and the like are also used.

In the present invention, an immunohistochemical method is also applicable.

"Western blotting" is a method for detecting a protein transferred on a membrane such as PVDF membrane and the like, using an antibody. Since a specific bond between antibody and antigen is utilized, only a small amount of a sample is required and a specific target protein can be detected.

By the "hybridization" is meant interaction between complementary nucleic acid strands. Since DNA has a double stranded structure based on the complementary interaction (C is always bonded to G and A is always bonded to T), when the complementary strands are separated, they preferably reanneal, or "hybridize" with each other. This also occurs between two DNA strands and between a DNA strand and an RNA strand, having sufficiently complementary base sequence. Hybridization occurs in all physiological reactions of DNA, such as replication, transcription and the like, and forms a basis of many molecular biological methods such as southern blotting, northern blotting, PCR and the like.

"PCR (Polymerase Chain Reaction)" means a reaction for specifically amplifying a DNA fragment sandwiched between one set of primers by performing DNA polymerase reactions continuously and as chain reactions. Of these, the "RT-PCR (reverse transcription-polymerase chain reaction) method" combining a reverse transcription reaction and PCR shows highest sensitivity.

The "ISH (in situ hybridization) method" is an effective means as a detection method of gene expression in tissue fragment. The FISH method is a combination of this method and a fluorescence detection method.

The "northern blotting" is a technique aiming at analysis of mRNA. It comprises electrophoresis of RNA, which easily takes a secondary structure, under degenerative conditions, and transfer thereof onto a membrane (nylon, nitrocellulose etc.). According to the degenerative method, there are 1. a method using formamide and formaldehyde, 2. a method using gyloxal, 3. a method using methylmercury hydroxide and the like.

The "southern blotting" is a method of transfer described by Southern in 1975, wherein a transcribed DNA region having a base sequence complementary to a labeled nucleic acid probe is detected.

For detection of expression or activity of Her2 and/or EGFR, a tissue (cancer tissue, blood vessel wall tissue, skin, oral mucosa etc.) or a body fluid (blood, lymph) and the like, which is obtained from patients, is applied to a test as recited in the above to detect expression or activity of Her2 and/or EGFR.

Specific examples of "a disease caused by overexpression or activation of Her2 and/or EGFR" include cancers such as brain tumor, pharyngeal cancer, laryngeal cancer, tongue cancer, esophageal cancer, gastric cancer, colorectal cancer, non-small cell lung cancer, pancreatic cancer, bile duct cancer, gallbladder cancer, liver cancer, renal cancer, bladder cancer, prostate cancer, breast cancer, ovarian cancer, cervical cancer, endometrial cancer, skin cancer, childhood solid cancer, bone tumor, hemangioma and the like, angiogenesis associated with diabetic retinopathy, arteriosclerosis, psoriasis and the like. Preferred are brain tumor, pharyngeal cancer, laryngeal cancer, tongue cancer, esophageal cancer, gastric cancer, colorectal cancer, non-small cell lung cancer, pancreatic cancer, bile duct cancer, gallbladder cancer, liver cancer, renal cancer, bladder cancer, prostate cancer, breast cancer, ovarian cancer, cervical cancer, endometrial cancer, skin cancer and the like and more preferred are brain tumor, gastric cancer, colorectal cancer, non-small cell lung cancer, pancreatic cancer, renal cancer, prostate cancer, breast cancer, ovarian cancer and the like.

The "overexpression or activation of Her2 and/or EGFR" is an expression or activity not less than the expression amount or activity necessary for homeostasis of living organisms, and the expression or activity not less than the expression amount or activity necessary for normal tissue of the same origin.

The "patients showing overexpression or activation of Her2 and/or EGFR" means the patients wherein at least one of Her2 and EGFR is excessively expressed or activated, and preferably the patients wherein both are excessively expressed or activated. The Her2 and/or EGFR inhibitor of the present invention is characterized by administration for the treatment of patients, wherein Her2 and/or EGFR are/is excessively expressed or activated as mentioned above.

The "Her2 and/or EGFR inhibitor" of the present invention is preferably a Her2 and EGFR inhibitor to be administered to patients wherein Her2 and EGFR are excessively expressed or activated, and may be a mixture of a Her2 inhibitor and an EGFR inhibitor. It is possible to use a Her2 inhibitor and an EGFR inhibitor simultaneously, separately or at time intervals. In other words, it is possible to administer a Her2 inhibitor and an EGFR inhibitor simultaneously, separately or, for example, in a staggered manner in a single day or at given time intervals for several days to several weeks or several months, by various different routes.

The Her2 inhibitor to be used in the present invention includes an anti-Her2 antibody Herceptin (Roche), TAK-165 (Takeda), ETH-102 (ExonHit Ther.) and the like, and the EGFR inhibitor to be used in the present invention includes Iressa (A. Zeneca), OSI-774 (Roche), PKI-166 (Novartis), EKB-569 (Wyeth) and the like. The production thereof and the like are described in WO02/06249, JP-A-2001-348385, JP-A-2002-69070, JP-A-9-136877, JP-A-11-60571 and the like for TAK-165, WO96/33980 and patent No. 3040486 for Iressa, WO96/30347 for OSI-774, WO97/02266 for PKI-166, and US Patent No. 6,002,008 for EKB-569.

When these are prepared into a mixture of a Her2 inhibitor and an EGFR inhibitor, one or more of respective

inhibitors are selected and a mixture thereof is produced by an appropriate known method and put to use.

## Brief Description of the Drawings

Fig. 1 shows a chemiluminescence taken with a luminoCCD camera in Example 4.

## Best Mode for Embodying the Invention

As the inhibitor of the present invention, a dual inhibitor is preferable. Specific examples thereof include an inhibitor that acts based on inhibition of the protein kinase activity by the enzyme, an inhibitor that acts by decreasing the Her2 and/or EGFR intracellular protein content, or an inhibitor of a physical interaction between Her2 and/or EGFR and a signal transduction molecule, and the like.

As examples of more specific compound of the "dual inhibitor", a substituted heteroaromatic compound represented by the following formula (I), which is disclosed in WO99/35146 (JP-T-2002-500225)

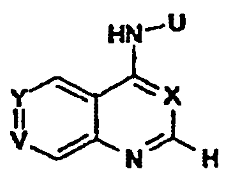

(I)

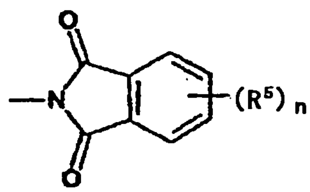

(a)

wherein X is N or CH; Y is $CR^1$ and V is N; or Y is N and V is $CR^1$; or Y is $CR^1$ and V is $CR^2$; or Y is $CR^2$ and V is $CR^1$; $R^1$ is $CH_3SO_2CH_2CH_2NHCH_2$-Ar- group (wherein Ar is selected from phenyl, furan, thiophene, pyrrole and thiazole, each of which is optionally substituted by 1 or 2 halogens, $C_{1-4}$ alkyl or $C_{1-4}$ alkoxy group on demand); $R^2$ is selected from the group consisting of hydrogen, halogen, hydroxy, $C_{1-4}$ alkyl, $C_{1-4}$ alkoxy, $C_{1-4}$ alkylamino and di [$C_{1-4}$ alkyl] amino ; U is phenyl, pyridyl, 3H-imidazolyl, indolyl, isoindolyl, indolinyl, isoindolinyl, 1H-indazolyl, 2,3-dihydro-1H-indazolyl, 1H-benzimidazolyl, 2,3-dihydro-1H-benzimidazolyl or 1H-benzotriazolyl group, each of which is substituted by $R^3$ group and optionally substituted on demand by at least one $R^4$ group independently selected; $R^3$ is selected from the group consisting of benzyl, halo-, dihalo- and trihalobenzyl, benzoyl, pyridylmethyl, pyridylmethoxy, phenoxy, benzyloxy, halo-, dihalo- and tribenzyloxy and benzenesulfonyl; or $R^3$ is trihalomethylbenzyl or trihalomethylbenzyloxy; or $R^3$ is a group of the above-mentioned formula (a) (wherein each $R^5$ is independently selected from halogen, $C_{1-4}$ alkyl and $C_{1-4}$ alkoxy; and n is 0-3) ; each $R^4$ is independently hydroxy, halogen, $C_{1-4}$ alkyl, $C_{2-4}$ alkenyl, $C_{2-4}$ alkynyl, $C_{1-4}$ alkoxy, amino, $C_{1-4}$ alkylamino, di [$C_{1-4}$ alkyl]amino, $C_{1-4}$ alkylthio, $C_{1-4}$ alkylsulfinyl, $C_{1-4}$ alkylsulfonyl, $C_{1-4}$ alkylcarbonyl, carboxy, carbamoyl, $C_{1-4}$ alkoxycarbonyl, $C_{1-4}$ alkanoylamino, N- ($C_{1-4}$ alkyl) carbamoyl, N, N-di ($C_{1-4}$ alkyl)carbamoyl, cyano, nitro or trifluoromethyl, particularly preferably, N-{3-chloro-4-[(3-fluorobenzyl)oxy]phenyl}-6-[5-({[2-(methanesulfonyl)ethyl]amino}methyl)-2-furyl]-4-quinazolinamine and the like, or N-[4-(3-chloro-4-fluorophenyl)amino]-7-[3-(4-morpholinyl)propoxy]quinazolin-6-yl]acrylamide and the like disclosed in WO00/31048 can be mentioned.

The compound of the formula (I) may take any form of a pharmaceutically acceptable salt, a hydrate or a solvate, a geometric isomer, an optical isomer or a racemate or a diastereomer mixture. As the pharmaceutically acceptable salt, salts with inorganic acids such as hydrochloric acid, hydrobromic acid, phosphoric acid, metaphosphoric acid, nitric acid and sulfuric acid and the like, salts with organic acids such as formic acid, acetic acid, tartaric acid, trifluoroacetic acid, citric acid, malic acid, lactic acid, fumaric acid, maleic acid, benzoic acid, phthalic acid, glycolic acid, glucuronic acid, gluconic acid, succinic acid, methanesulfonic acid, p-toluenesulfonic acid, isethionic acid and the like, salts with alkali metals or alkaline earth metals such as sodium, potassium, magnesium, calcium and the like, salts with organic bases such as ammonium, tetramethylamine, triethylamine, benzylamine, phenethylamine, monoethanolamine, diethanolamine, tris(hydroxyethylamine) and the like, salts with amino acids such as lysin, arginine, aspartic acid, glutamic acid and the like can be mentioned.

The production method and dual inhibitory action of the compound of the formula (I) are described in WO99/35146, and the production method and dual inhibitory action of N-[4-(3-chloro-4-fluorophenyl)amino-7-[3-(4-morpholinyl)propoxy]quinazolin-6-yl]acrylamide are described in WO00/31048.

The dual inhibitor of the present invention contains any of the above-mentioned compounds and other compounds having a dual inhibitory action (e.g., compound described in WO02/066451).

When the inhibitor of the present invention is used as a pharmaceutical agent, the inhibitor of the present invention is mixed with a pharmaceutically acceptable carrier (excipient, binder, disintegrant, corrigent, flavor, emulsifier, diluent, dissolution aids etc.) and can be administered orally or parenterally in the form of the obtained pharmaceutical composition or preparation (tablet, pill, capsule, granule, powder, syrup, emulsion, elixir, suspension, solution, injection, infusion, suppository etc.).

In the present invention, parenteral includes subcutaneous injection, intravenous injection, intramuscular injection, intraperitoneal injection, infusion method and the like.

As a dosage form of a solid administration for oral administration, those mentioned above such as powder, granule, tablet, pill, capsule and the like can be mentioned. In such dosage form, the active ingredient compound can contain at least one pharmaceutically acceptable additive (inert diluent, lubricant, preservative, antioxidant, disintegrant, binder, thickener, buffer, sweetener, flavor, perfume etc.).

As the liquid for oral administration, pharmaceutically acceptable emulsion, syrup, elixir, suspension, solution and the like can be mentioned, which may contain an inert diluent usually employed in the pertinent field.

A preparation for injection (sterile aqueous suspension or oil suspension for injection etc.) can be prepared by a method known in the pertinent field and using a suitable dispersing agent, wetting agent, suspending agent and the like. The sterile preparation for injection may be a sterile injectable solution or suspension using a diluent or solvent. In addition, a sterile involatile oil can be generally used as a solvent or suspending solvent. For this end, any involatile oil or fatty acid can be used.

The suppository for rectal administration can be produced by mixing the drug and a suitable non-stimulant excipient, such as one that is solid at ordinary temperature, liquid at the temperature of intestine, and melts in rectal to release the drug, and the like.

The dose is determined in consideration of the age, body weight, general health conditions, sex, diet, administration time, administration method, excretion speed, combination of drugs, level of disease for which the patient is under treatment or other factors. For example, when the above-mentioned compound represented by the formula (I) is used, the daily dose thereof varies depending on the condition and body weight of the patients, kind of the compound, administration route and the like. The compound is orally administered in a dose of 0.01-1000 mg/kg body weight/day, preferably 0.05-500 mg/kg body weight/day, which is once to several times a day. Parenterally, about 0.01-50 mg/kg body weight/day, preferably 0.01-20 mg/kg body weight/day, is preferably administered subcutaneously, intravenously, intramuscularly or rectally.

The prophylactic and/or therapeutic agent of the present invention to be used for patients who showed expression or activation of Her2 and/or EGFR is preferably provided as a pharmaceutical product by packaging with a written matter stating that the agent can or should be used for the patients determined to be suffering from a disease caused by expression or activation of Her2 and/or EGFR as a result of a diagnosis of the presence of expression or activity of Her2 and/or EGFR based on a test for detecting the expression or activity of Her2 and/or EGFR.

## Examples

The present invention is explained in more detail by illustrating Examples in the following, which are not to be construed as limitative unless they go beyond the gist of the invention.

### Example 1

EGFR tyrosine kinase inhibitory activity

(method)

The pharmaceutical agent PD 0183805 used for the test is known to inhibit EGFR tyrosine kinase and shows an in vivo antitumor effect on EGFR overexpression cancer A431[1)]. In addition, PD 0183805 dihydrochloride CI-1033 has been reported to inhibit tyrosine phosphorylation of Her2, erbB3 and erbB4 when MDA-MB-453 cells are stimulated with Heregulin[2)].

In the following description, PD 0183805 is abbreviated as PD, and PD 0183805 dihydrochloride CI-1033 is abbreviated as PD·2HCl. The chemical name and chemical structure of PD is as follows.

N-[4-(3-chloro-4-fluorophenyl)amino-7-[3-(4-morpholinyl)propoxy]quinazolin-6-yl-]acrylamide

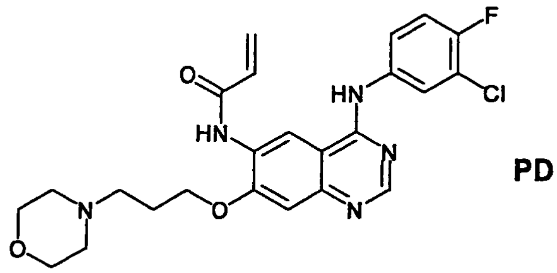

PD

In addition, PD and PD·2HCl was synthesized according to the method described in WO00/31048.

1) Vincent, P.W., Patmore, S.J., Bridges, A.J., Kirkish, L.S., Dudeck, R.C., Leopold, W.R., Zhou, H., Elliott, W.L. Proc. Am. Assoc. Cancer Res., 40: 117, 1999.
2) Slichenmyer, W.J., Elliott, W.L. and Fry, D.W. Semin. Oncol., 28: 80, 2001.

The test was performed using partially purified EGFR obtained from human epidermoid cancer cell A431 (provided by Cell Resource Center for Biomedical Research, the Institute of Development, Aging and Cancer, Tohoku University; catalog No. TKG-0182, or when purchased from ATCC; ATCC No. CRL-1555) according to a modification of the tyrosine kinase activity assay method of Linda J. Pike et al. (Proceedings of the National Academy of Science of the U.S.A., 1982, 79, 1433). The method was detailedly as follows.

A431 cells were incubated in a 10% FBS supplemented DMEM medium at 37°C under 5% carbon dioxide, and the cells were homogenized in a solution containing 10 mM HEPES buffer (pH 7.4), 0.25 M sucrose and 0.1 mM EDTA, after which centrifuged at 3,000×G for 5 min. The supernatant was centrifuged at 100,000×G for 30 min to give an A431 cell membrane fraction, which was subjected to the assay as the partially purified EGFR as an enzyme source.

To a reaction mixture (final concentration 1% DMSO) of the pharmaceutical agent dissolved in dimethyl sulfoxide (DMSO), the A431 cell membrane fraction (10-15 μg), 30 mM HEPES buffer (pH 7.4), 2 mM $MnCl_2$ and 100 μM $Na_3VO_4$ was added 100 ng of EGF and synthetic substrate angiotensin II (Asp-Arg-Val-Tyr-Ile-His-Pro-Phe, 50 μg) and adenosine triphosphate (containing 37 KBq of $\gamma$-$^{32}$P-labeled form, final concentration 10 μM) were further added to start the reaction. The volume then was 60 μL.

The reaction was carried out in ice for 30 min and stopped by the addition of 10 mg/mL bovine serum albumin (6 μL) and 20% trichloroacetic acid (25 μL). The reaction mixture was left standing in the ice for 30 min.

After centrifugation at 5000G for 2 min, the supernatant was sampled (40 μL) and allowed to adsorb onto P81 phosphocellulose paper. These were immersed in 0.75% aqueous phosphoric acid for 5 min for washing and this washing operation was repeated 4 times. The paper was taken out and measured for $^{32}$P count on a liquid scintillation counter, and the obtained value was taken as A.

A reaction without a pharmaceutical agent and a reaction without both a pharmaceutical agent and EGF were simultaneously measured for $^{32}$P count, and the obtained values were taken as B and C, respectively.

From these values, the tyrosine kinase inhibitory rate can be determined from the following formula:

$$\text{Inhibitory rate (\%)} = 100-\{(A-C)/(B-C)\}\times 100$$

$IC_{50}$ (50% inhibitory concentration) was calculated from the inhibitory rates obtained by changing the addition concentration of the pharmaceutical agent. The results are shown in the following table.

Table 1

| EGFR tyrosine kinase inhibitory activity | |
|---|---|
| pharmaceutical agent | $IC_{50}$ nM |
| PD | 0.4 |

**Example 2**

Cellular Her2 tyrosine kinase inhibitory activity

(method)

As the cells, NIH3T3 mouse fibroblast cell line (provided by Cell Resource Center for Biomedical Research, the Institute of Development, Aging and Cancer, Tohoku University; catalog No. TKG-0298) transformed with mutant c-erbB2 constitutively activated by substituting 659th valine by glutamic acid (hereinafter to be referred to as A4 cell) was used. This cell line was cultured and maintained in a 10% FBS supplemented DMEM/F12 mixed medium (hereinafter to be referred to as an assay medium) in a plastic dish at 37°C under 5% carbon dioxide.

A4 cells suspended in an assay medium were seeded in a 12 well plate at $3\times10^5$/well, and the cells that reached confluent were incubated with a pharmaceutical agent at 37°C for 2 hr. The cells were washed once with PBS and re-suspended in cell lysis buffer (60 mM Tris (pH 6.8), 2% SDS, 10% glycerol, 5% betamercaptoethanol, 0.001% bromophenol blue) and ultrasonicated, then used as a whole cell lysate for western blotting.

The whole cell lysate corresponding to the protein amount of 25 μg was subjected to 7.5% SDS-polyacrylamide gel electrophoresis, and then transferred onto a PVDF membrane. The membrane was blocked, and incubated with anti-phosphotyrosine mouse monoclonal antibody PY20 (Transduction Laboratories, catalog No. P11120) in a 0.1% Tween 20 added Tris buffer, after which treated with HRP labeled anti-mouse secondary antibody (DAKO, catalog No. P0447). The membrane was treated with chemiluminescent reagent ECL western blotting detection reagents (Amersham Pharmacia Biotech, catalog No. RPN2209) and the chemiluminescence was photographed with a luminoCCD camera. The photographing of the chemiluminescence and image analysis were performed using Densitograph for Macintosh (ATTO, product type AE-6930).

The obtained phosphorylation signals were quantified and evaluated for phosphorylation inhibition by the pharmaceutical agent in % control, wherein the signal without addition of the compound was 100% control and the background signal was 0%. The results are shown in the following Table.

(Table 2)

| cellular Her2 tyrosine kinase inhibitory activity | | |
|---|---|---|
| pharmaceutical agent | % control (0.1 μM) | % control (1 μM) |
| PD | 77 | 12 |

From the results of Examples 1, 2, it is recognized that PD shows an inhibitory activity on both Her2 and EGFR, namely, PD acts as a Her2 and/or EGFR inhibitor.

**Example 3**

in vivo antitumor effects

(method)

Human pancreatic cancer HPAC (ATCC No. CRL-2119), human colorectal cancer LS174T (ATCC No. CL-188) and human lung cancer NCI-H520 (ATCC No. HTB-182) were purchased from ATCC. Human cervical cancer ME180 was supplied by Cell Resource Center for Biomedical Research, the Institute of Development, Aging and Cancer, Tohoku University (catalog No. TKG-0437, when purchased from ATCC, ATCC No. HTB-33). Cultured human cancer cells suspended in PBS were implanted subcutaneously in the back of female Balb/c nude mice (Balb/cAJcl-nu mouse, CLEA Japan Inc., 5-week-old when received) at $5\times10^6$/100 μl. When about 7 days later and the average volume of the implanted tumors almost reached 100 $mm^3$, the mice were allotted by 4 mice per group such that the average tumor volume of each group became equal.

For the tumor volume, the longer diameter and the shorter diameter were measured with calipers and calculated as [(shorter diameter)$^2\times$(longer diameter/2)]=tumor volume [$mm^3$]. A pharmaceutical agent was administered by oral gavage once a day for 14 consecutively days from the day of allottment, and the pharmaceutical agent was not administered to the mice of the control group. The relative tumor growth rate was calculated based on the tumor volume on the administration start day for the pharmaceutical agent treatment group against the control group as rate 1. The antitumor effects are shown in % control. The % control was calculated by the following formula.

% control =[(average relative tumor growth rate of pharmaceutical agent treatment group on the final day-1)/(average relative tumor growth rate of control group on the final day-1)]×100

The results are shown in the following Table.

(Table 3)

| Antitumor effects on human pancreatic cancer HPAC (both EGFR, Her2 positive) | | | |
|---|---|---|---|
| pharmaceutical agent | dose mg/kg | average relative tumor growth rate | % control |
| control | - | 4.6 | 100 |
| PD | 10 | 2.4 | 39 |
| PD | 30 | 1.4 | 11 |

(Table 4)

| Antitumor effects on human cervical cancer ME180 (EGFR positive, Her2 negative) | | | |
|---|---|---|---|
| pharmaceutical agent | dose mg/kg | average relative tumor growth rate | % control |
| Control | - | 10.9 | 100 |
| PD·2HCl | 10 | 3.5 | 25 |
| PD·2HCl | 30 | 2.7 | 17 |

(Table 5)

| Antitumor effects on human rectal cancer LS174T (EGFR negative, Her2 positive) | | | |
|---|---|---|---|
| pharmaceutical agent | dose mg/kg | average relative tumor growth rate | % control |
| control | - | 18.2 | 100 |
| PD | 10 | 10.7 | 56 |
| PD | 30 | 6.8 | 34 |

(Table 6)

| Antitumor effects on human lung cancer NCI-H520 (both EGFR, Her2 negative) | | | |
|---|---|---|---|
| pharmaceutical agent | dose mg/kg | average relative tumor growth rate | % control |
| Control | - | 8.7 | 100 |
| PD·2HCl | 10 | 8.3 | 95 |
| PD·2HCl | 30 | 7.7 | 87 |

From the results shown in above Tables, it is recognized that a Her2 and/or EGFR inhibitor shows a growth suppressive effect on cancer cells of (both EGFR, Her2 positive), (EGFR positive, Her2 negative) or (EGFR negative, Her2 positive), namely, that a Her2 and/or EGFR inhibitor is effective for the prophylaxis or treatment of a disease caused by overexpression or activation of Her2 and/or EGFR.

**Example 4**

Confirmation of expression of EGFR or Her2 by Western blotting

(method)

Human pancreatic cancer HPAC (ATCC No. CRL-2119), PANC1 (ATCC No. CRL-1469), human lymphoma Daudi (ATCC No. CCL-213) and human colorectal cancer LS174T (ATCC No. CL-188) were purchased from ATCC. human vulvar cancer A431 (catalog No. TKG-0182), human cervical cancer ME180 (catalog No. TKG-0437), human tongue cancers HSC-3 (catalog No. TKG-0484) and HSC-4 (catalog No. TKG-0489) were provided by Cell Resource Center for Biomedical Research, the Institute of Development, Aging and Cancer, Tohoku University.
The cells were cultured in a 100 mm culture dish until they became almost confluent. The medium in the 100 mm culture dish were removed and washed once with PBS. Then, about 0.6 mL of RIPA buffer (50 mM Tris (pH 7.4), 150 mM sodium chloride, 1% NP-40, 0.25% deoxycholic acid, 1 mM EDTA and protease inhibitor cocktail) was added and the mixture was stood on ice for 10 min. The cell lysate was recovered in a 1.5 mL centrifugation tube and centrifuged at 10,000 rpm for 10 min with cooling. The supernatant was transferred to a different tube and used for western blotting.
The cell lysate corresponding to the protein amount of 25 μg was subjected to 7.5% SDS-polyacrylamide electrophoresis, and then transferred onto a PVDF membrane. The membrane was blocked, and incubated with a specific antibody against EGFR (Santa Cruz Biotechnology, catalog No. sc-03) or a specific antibody against Her2 (Upstate biotechnology, catalog No. 06-562) in a 0.1% Tween 20 added Tris buffer together, after which treated with HRP labeled anti-rabbit secondary antibody (ICN Pharmaceuticals, catalog No. 55689). The membrane was treated with chemiluminescent reagent ECL western blotting detection reagents (Amersham Pharmacia Biotech, catalog No. RPN2209) and the chemiluminescence was photographed with a luminoCCD camera.
The images are shown in Fig. 1. In addition, explanation of each lane in Fig. 1 is shown in the following Table.

(Table 7)

| lane | cell line | upper column | lower column |
|---|---|---|---|
| | | EGFR expression | Her2 expression |
| 1 | HPAC | positive | positive |
| 2 | PANC1 | positive | positive |
| 3 | ME180 | positive | negative |
| 4 | Daudi | negative | negative |
| 5 | A431 | positive | positive |
| 6 | LS174T | negative | positive |
| 7 | HSC3 | positive | positive |
| 8 | HSC4 | positive | positive |

**Example 5**

Confirmation of expression of EGFR or Her2 by RT-PCR method

(method)

The test was performed according to Molecular Cloning, A Laboratory Manual Vol. 1, Second Edition. The detail is given in the following.
Total RNA was extracted from cancer cells with S.N.A.P. ™ total RNA isolation kit (invitrogen, catalog No. 45-0472). 1 μg of the obtained total RNA was used for reverse transcription reaction. The reverse transcription reaction was performed using 1st Strand cDNA Synthesis Kit for RT-PCR (Roche, catalog No. 1 483 188).
As the primer used then was random primer p(dN)6. 1 μL of the obtained cDNA was used to perform PCR. For detection of EGFR mRNA, Human Epidermal growth factor receptor and/or GAPDH genes Dual-PCR kit (Maxim Biotech, catalog No. DP-10065-G) attached primer was used as a primer and TaKaRa Ex Taq™b TAKARA SHUZO CO., LTD., catalog No. RR001A) was used as an enzyme. The reaction conditions were 1 cycle of 96°C 1 minute, 30 cycles of 96°C 1 minute and 58°C 1 minute 30 seconds, and 1 cycle of 72°C 10 minutes. For detection of Her2 mRNA, RT-PCR primer set HUMAN erb-B2 (CLP, catalog No. 5254.H)attached primer was used as a primer and TaKaRa Ex Taq™ (TAKARA SHUZO CO., LTD., catalog No. RR001A) was used as an enzyme. The reaction conditions were 1

cycle of 94°C 5 minutes and 60°C 5 minutes, 40 cycles of 72°C 2 minutes, 94°C 1 minute and 60°C 1 minute, and 1 cycle of 72°C 10 minutes. The obtained PCR product was subjected to agarose electrophoresis for confirmation of the expression.

From the results of the above-mentioned Examples 4, 5, it is appreciated that confirmation of the expression of Her2 and/or EGFR is achieved by western blotting or RT-PCR method.

From the results of the above-mentioned Examples 2, 4, moreover, it is appreciated that the test of Her2 or EGFR activation is possible. Using such a test method, the expression or activity of Her2 and/or EGFR in patients is tested, and when overexpression or activation of Her2 and/or EGFR are/is diagnosed, patient is evaluated as suffering from a disease caused by the expression or activation of Her2 and/or EGFR, and the pharmaceutical product of the present invention is administered.

## Industrial Applicability

As mentioned above, the inhibitor of the present invention is an effective treatment method of cancer patients and also expected to be an agent for the prophylaxis and/or treatment of preventing transition from hormone sensitive cancer to resistant cancer in prostate cancer and breast cancer. Moreover, it is expected to an agent for the prophylaxis and/or treatment of angiogenesis associated with the growth of solid cancer and sarcoma, angiogenesis associated with cancer metastasis, angiogenesis associated with diabetic retinopathy, arteriosclerosis, psoriasis and the like.

This application is based on a patent application No. 162130/2002 filed in Japan, the contents of which are hereby incorporated by reference.

## Claims

1. A Her2 and/or EGFR inhibitor to be administered to a subject determined to show overexpression or activation of Her2 and/or EGFR as a result of a diagnosis of the subject for the expression or activity of Her2 and/or EGFR based on a test for detecting the expression or activity of Her2 and/or EGFR.

2. The inhibitor of claim 1 to be administered to a subject determined to show activation of Her2 and/or EGFR as a result of a diagnosis of the subject for the activity of Her2 and/or EGFR based on a test for detecting the activity of Her2 and/or EGFR.

3. The inhibitor of claim 1 to be administered to a subject determined to show overexpression or activation of Her2 and EGFR as a result of a diagnosis of the subject for the expression or activity of Her2 and EGFR based on a test for detecting the expression or activity of Her2 and EGFR.

4. The inhibitor of claim 3 to be administered to a subject determined to show activation of Her2 and EGFR as a result of a diagnosis of the subject for the activity of Her2 and EGFR based on a test for detecting the activity of Her2 and EGFR.

5. The inhibitor of any of claims 1 to 4, wherein the subject is a patient expected to suffer from a disease caused by overexpression or activation of Her2 and/or EGFR.

6. The inhibitor of any of claims 1 to 4, wherein the subject is a patient expected to suffer from a disease caused by overexpression or activation of Her2 and EGFR.

7. The inhibitor of any of claims 1 to 6, wherein the subject is a human.

8. The inhibitor of any of claims 1 to 4, wherein the test for detecting the expression or activity of Her2 and/or EGFR is an extracorporeal test.

9. The inhibitor of any of claims 1 to 4, wherein the test for detecting the expression or activity of Her2 and EGFR is an extracorporeal test.

10. The inhibitor of claim 3, which is a mixture of a Her2 inhibitor and an EGFR inhibitor.

11. The inhibitor of any of claims 1 to 9, which is used for administering a Her2 inhibitor and/or an EGFR inhibitor simultaneously, separately or at time intervals.

**12.** The inhibitor of claim 8 or 9, wherein the extracorporeal test is an immunological method using an antibody, or a hybridization method using a nucleic acid and a nucleic acid derivative.

**13.** The inhibitor of claim 12, wherein the immunological method using an antibody is selected from the group consisting of an enzyme-linked immunosorbent assay, an enzyme-linked immunoassay, a radioimmunoassay, an immunohistochemical method and western blotting.

**14.** The inhibitor of claim 12, wherein the hybridization method using a nucleic acid and a nucleic acid derivative is selected from the group consisting of an RT-PCR method, an ISH method, a FISH method, northern blotting and southern blotting method.

**15.** The inhibitor of any of claims 1 to 14, which is a substituted heteroaromatic compound represented by the following formula (I)

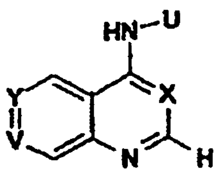

(I)

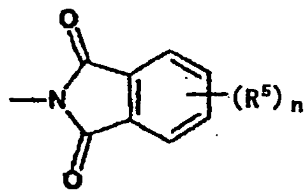

(a)

wherein X is N or CH; Y is $CR^1$ and V is N; or Y is N and V is $CR^1$; or Y is $CR^1$ and V is $CR^2$; or Y is $CR^2$ and V is $CR^1$; $R^1$ is $C_{1-4}$ alkyl, $C_{1-4}$ alkoxy, $CH_3SO_2CH_2CH_2NHCH_2$-Ar- (wherein Ar is selected from phenyl, furan, thiophene, pyrrole and thiazole, each of which is optionally substituted by 1 or 2 halogens, $C_{1-4}$ alkyl or $C_{1-4}$ alkoxy on demand) or -C=C-C ($R^6$) ($R^7$) ($R^8$) (wherein $R^6$, $R^7$ and $R^8$ are each independently a hydrogen atom, hydroxy, halogen, $C_{1-4}$ alkyl or $C_{1-4}$ alkoxy, or $C_{3-6}$ cycloalkyl wherein the ring is optionally substituted by hydrogen atom or $C_{1-4}$ alkyl and optionally contains 1 or 2 hetero atoms selected from O, S and N therein; $R^2$ is selected from the group consisting of hydrogen, halogen, hydroxy, $C_{1-4}$ alkyl, $C_{1-4}$ alkoxy, $C_{1-4}$ alkylamino, di [$C_{1-4}$ alkyl]amino and -NHCO-$R^9$ (wherein $R^9$ is $C_{1-4}$ alkyl, $C_{1-4}$ alkoxy, $C_{2-4}$ alkenyl or $C_{2-4}$ alkynyl) ; U is phenyl, pyridyl, 3H-imidazolyl, indolyl, isoindolyl, indolinyl, isoindolinyl, 1H-indazolyl, 2,3-dihydro-1H-indazolyl, 1H-benzimidazolyl, 2,3-dihydro-1H-benzimidazolyl or 1H-benzotriazolyl group, each of which is substituted by $R^3$ group and optionally substituted on demand by at least one $R^4$ group selected independently; $R^3$ is selected from the group consisting of benzyl, halo-, dihalo- and trihalobenzyl, benzoyl, pyridylmethyl, pyridylmethoxy, phenoxy, benzyloxy, halo-, dihalo- and tribenzyloxy and benzenesulfonyl; or $R^3$ is trihalomethylbenzyl or trihalomethylbenzyloxy; or $R^3$ is a group of the above-mentioned formula (a) (wherein each $R^5$ is independently selected from halogen, $C_{1-4}$ alkyl and $C_{1-4}$ alkoxy; and n is 0-3); each $R^4$ is independently hydroxy, halogen, $C_{1-4}$ alkyl, $C_{2-4}$ alkenyl, $C_{2-4}$ alkynyl, $C_{1-4}$ alkoxy, amino, $C_{1-4}$ alkylamino, di [$C_{1-4}$ alkyl] amino, $C_{1-4}$ alkylthio, $C_{1-4}$ alkylsulfinyl, $C_{1-4}$ alkylsulfonyl, $C_{1-4}$ alkylcarbonyl, carboxy, carbamoyl, $C_{1-4}$ alkoxycarbonyl, $C_{1-4}$ alkanoylamino, N- ($C_{1-4}$ alkyl) carbamoyl, N,N-di ($C_{1-4}$ alkyl) carbamoyl, cyano, nitro or trifluoromethyl, or a pharmaceutically acceptable salt thereof, a hydrate or solvate thereof, an optically active substance or a racemate thereof, or a mixture of diastereomers thereof.

**16.** The inhibitor of any of claims 1 to 15, which is (4-(3-fluorobenzyloxy)-phenyl)-(6-(5-((2-methanesulfonylethylamino) methyl)-furan-2-yl)-pyrido[3,4-d]pyrimidin-4-yl)-amine;
(4-benzyloxyphenyl)-(6-(5-((2-methanesulfonyl-ethylamino)-methyl)-furan-2-yl)-quinazolin-4-yl)-amine;
N-{4-[(3-fluorobenzyl)oxy]phenyl}-6-[5-({[2-(methylsulfonyl)ethyl]amino}methyl)-2-furyl]-4-quinazolinamine;
N-[4-(benzyloxy)phenyl]-7-methoxy-6-[5-({[2-(methylsulfonyl)ethyl]amino}methyl)-2-furyl]-4-quinazolinamine;
N-(1-benzyl-1H-indazol-5-yl)-7-methoxy-6-[5-({[2-(methylsulfonyl)ethyl]amino}methyl)-2-furyl]-4-quinazolinamine;
N-{3-fluoro-4-[(3-fluorobenzyl)oxy]phenyl}-6-[5-({[2-(methylsulfonyl)ethyl]amino}methyl)-2-furyl]-4-quinazolinamine;
N-[1-(3-fluorobenzyl)-1H-indazol-5-yl]-6-,[2-({[2-(methylsulfonyl)ethyl]amino}methyl)-1,3-thiazol-4-yl]-4-quinazolinamine;
6-[5-({[2-(methylsulfonyl)ethyl]amino}methyl)-2-furyl]-N-[4-(phenylsulfonyl)phenyl]-4-quinazolinamine;
N-{3-fluoro-4-[(3-fluorobenzyl)oxy]phenyl}-6-[2-({[2-(methylsulfonyl)ethyl]amino}methyl)-1,3-thiazol-4-yl]-4-quinazolinamine;
N-(1-benzyl-1H-indazol-5-yl)-6-[2-({[2-(methylsulfonyl)ethyl]amino}methyl)-1,3-thiazol-4-yl]-4-quinazolinamine;
N-(3-fluoro-4-benzyloxyphenyl)-6-[5-({[2-(methylsulfonyl)ethyl]amino}methyl)-4-furyl]-4-quinazolinamine;

N-(3-chloro-4-benzyloxyphenyl)-6-[2-({[2-(methylsulfonyl)ethyl]amino}methyl)-4-furyl]-4-quinazolinamine;
N-{3-chloro-4-[(3-fluorobenzyl)oxy]phenyl}-6-[5-({[2-(methylsulfonyl)ethyl]amino}methyl)-2-furyl]-4-quinazolinamine;
N-(1-benzyl-1H-indazol-5-yl)-7-fluoro-6-[5-({[2-(methylsulfonyl)ethyl]amino}methyl)-2-furyl]-4-quinazolinamine;
N-(3-trifluoromethyl-4-benzyloxyphenyl)-6-[5-({[2-(methylsulfonyl)ethyl]amino}methyl)-4-furyl]-4-quinazolinamine;
N-[4-(3-chloro-4-fluorophenyl)amino-7-[3-(4-morpholinyl)propoxy]quinazolin-6-yl]acrylamide;
N-{4-[(3-chloro-4-fluorophenyl)amino]-7-[3-methyl-3-(4-methyl-1-piperazinyl)-1-butynyl]-6-quinazolinyl}acrylamide; or
N-{3-chloro-4-[(3-fluorobenzyl)oxy]phenyl}-6-[5-({[2-(methanesulfonyl)ethyl]amino}methyl)-2-furyl]-4-quinazolinamine, or a pharmaceutically acceptable salt thereof, a hydrate or a solvate thereof, an optically active substance or a racemate thereof, or a mixture of diastereomers thereof.

**17.** The inhibitor of any of claims 1 to 16, which is N-[4-(3-chloro-4-fluorophenyl)amino]-7-[3-(4-morpholinyl)propoxy] quinazolin-6-yl]acrylamide, or N-{3-chloro-4-[(3-fluorobenzyl)oxy]phenyl}-6-[5-({[2-(methanesulfonyl)ethyl]amino} methyl)-2-furyl]-4-quinazolinamine or a pharmaceutically acceptable salt thereof, a hydrate or a solvate thereof, an optically active substance or a racemate thereof, or a mixture of diastereomers thereof.

**18.** A pharmaceutical composition comprising an inhibitor of any of claims 1 to 17 as an active ingredient and a pharmaceutically acceptable carrier.

**19.** The pharmaceutical composition of claim 18, which is an agent for the prophylaxis and/or treatment of a disease caused by overexpression or activation of Her2 and/or EGFR.

**20.** The pharmaceutical composition of claim 19, wherein the disease caused by the overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis.

**21.** An agent for the prophylaxis and/or treatment of a disease caused by overexpression or activation of Her2 and/or EGFR, which is to be administered to a subject determined to show overexpression or activation of Her2 and/or EGFR as a result of a diagnosis of the subject for the expression or activity of Her2 and/or EGFR based on a test for detecting the expression or activity of Her2 and/or EGFR.

**22.** The agent of claim 21, wherein the disease caused by overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis.

**23.** A method for the prophylaxis and/or treatment of a disease caused by overexpression or activation of Her2 and/or EGFR, which comprises administering an effective dose of a Her2 and/or an EGFR inhibitor to a subject determined to show overexpression or activation of Her2 and/or EGFR as a result of a diagnosis of the subject for the expression or activity of Her2 and/or EGFR based on a test for detecting the expression or activity of Her2 and/or EGFR.

**24.** The method of claim 23, wherein the disease caused by overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis.

**25.** A commercial package comprising the pharmaceutical composition of any of claims 18 to 20 and a written matter associated therewith, the written matter stating that the pharmaceutical composition can or should be used for the prophylaxis and/or treatment of a disease caused by overexpression or activation of Her2 and/or EGFR.

**26.** The commercial package of claim 25, wherein the disease caused by overexpression or activation of Her2 and/or EGFR is cancer, angiogenesis associated with the growth of cancer or sarcoma, angiogenesis associated with cancer metastasis, angiogenesis associated with diabetic retinopathy, arteriosclerosis or psoriasis.

FIG. 1

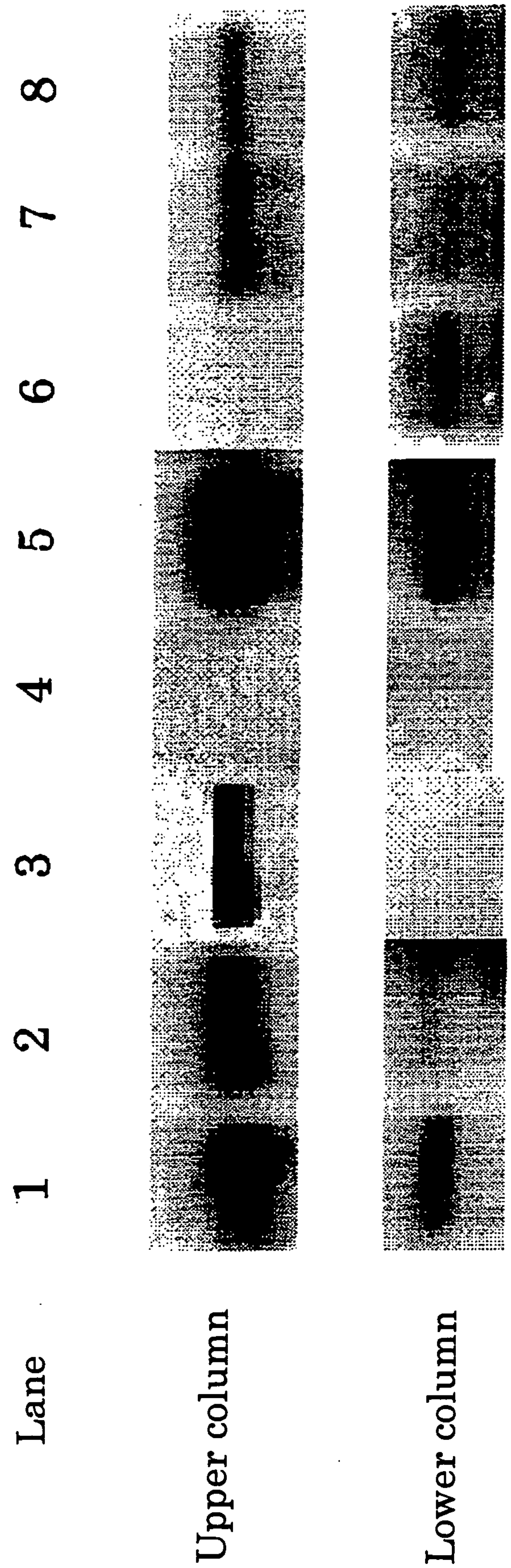

## INTERNATIONAL SEARCH REPORT

International application No.
PCT/JP03/06988

**A. CLASSIFICATION OF SUBJECT MATTER**

Int.Cl[7] A61K45/00, 31/517, 31/519, 31/5377, A61P9/10, 17/06, 27/02, 35/00

According to International Patent Classification (IPC) or to both national classification and IPC

**B. FIELDS SEARCHED**

Minimum documentation searched (classification system followed by classification symbols)

Int.Cl[7] A61K45/00, 31/517, 31/519, 31/5377, A61P9/10, 17/06, 27/02, 35/00

Documentation searched other than minimum documentation to the extent that such documents are included in the fields searched

Electronic data base consulted during the international search (name of data base and, where practicable, search terms used)

CA(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X | WO 99/35146 A1 (Glaxo Group Ltd.), 15 July, 1999 (15.07.99), Full text & CA 2317589 A & AU 749549 B2 & BR 9906904 A & EP 1047694 A1 & JP 2002-500225 A | 1-22,25-26 |
| X | WO 00/31048 A1 (Warnar-Lambert Co.), 02 June, 2000 (02.06.00), Full text & EP 1131304 B1 & US 6344455 B1 & JP 2002-530386 A | 1-22,25-26 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 August, 2003 (12.08.03) | 26 August, 2003 (26.08.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06988

**Box I Observations where certain claims were found unsearchable (Continuation of item 2 of first sheet)**

This international search report has not been established in respect of certain claims under Article 17(2)(a) for the following reasons:

1. [X] Claims Nos.: 23-24
   because they relate to subject matter not required to be searched by this Authority, namely:
   Claims 23 to 24 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)